# EUROPÄISCHE PATENTANMELDUNG

(11) **EP 3 957 339 A1**
(43) Veröffentlichungstag der Anmeldung: **23.02.2022**
(21) Anmeldenummer: 21191538.4
(22) Anmeldetag: 16.08.2021
(51) Int. Cl.: A61L 27/04, A61L 27/58, B33Y 10/00, C22C 38/04

(54) **IMPLANTATWERKSTOFF UND DESSEN VERWENDUNG**

(30) Priorität: 19.08.2020 DE 102020121729
(71) Anmelder: Leibniz-Institut für Festkörper- und Werkstoffforschung Dresden e.V., 01069 Dresden (DE)
(72) Erfinder: Hufenbach, Julia, 01069 Dresden (DE); Otto, Martin, 01069 Dresden (DE); Paul, Birgit, 01069 Dreden (DE); Kühn, Uta, 01069 Dresden (DE); Rößler, Ulrich, 01069 Dresden (DE); Gebert, Annett, 01069 Dresden (DE)
(74) Vertreter: Kailuweit & Uhlemann Patentanwälte Partnerschaft mbB

(57) **Zusammenfassung**

Die Erfindung betrifft einen Implantatwerkstoff umfassend eine biologisch abbaubare Eisenbasislegierung mit einer Zusammensetzung gemäß Formel (I) Fe_{100-b-c-d-e-f}Mn_{b}C_{c}X_{d}YₑZ_{f} (I), wobei X = Cu, und wobei Y = Zn, und wobei Z = mindestens eines der Elemente Mg, Ca, S sind, und wobei die Eisenbasislegierung mindestens eines der Elemente X, Y und Z enthält, undwobei 10 ≤ b ≤ 40 und 0,1 ≤ c ≤ 1,5 und 0 ≤ d ≤ 20 und 0 ≤ e ≤ 30 und 0 ≤ f ≤ 20 (b, c, d, e, f in Massenanteilen) und wobei die Eisenbasislegierung mindestens das Element X enthält und 3 < d ≤ 20 ist, oder dass die Eisenbasislegierung mindestens das Element Y und/oder Z enthält

## Beschreibung

Die Erfindung betrifft einen Implantatwerkstoff und dessen Verwendung.

Implantatwerkstoffe haben eine Vielzahl von Anforderungen zu erfüllen, bspw. die chemische, biologische und mechanische Kompatibilität mit dem zu ersetzenden bzw. zum umliegenden Gewebe. Gerade im Bereich temporärer Implantate spielt die Biodegradation eine wichtige Rolle, um u.a. Folgeeingriffe zur Entfernung des Implantats zu vermeiden. Biodegradation meint dabei elektrochemische, chemische, hydrolytische, enzymatische und andere stoffwechselbedingte Abbauprozesse im lebenden Organismus, die vor allem durch den Kontakt des biodegradierbaren Implantatwerkstoffs mit Körperflüssigkeiten verursacht werden und zu einer allmählichen Auflösung des Implantats führen. Je nach Art des Implantats und dessen Anwendung im Organismus werden unterschiedliche Anforderungen an die Biodegradation gestellt.

Biodegradierbare Implantatwerkstoffe für temporäre Implantate aus Legierungen auf Eisen-, Magnesium- und Zinkbasis mit guter chemischer, biologischer und mechanischer Kompatibilität sind aus [1] bekannt.

Reines Zink und dessen Legierungen als temporäre Implantatwerkstoffe weisen moderate Abbauraten in biologischen Systemen auf. Nachteilig bei Zink und dessen Legierungen sind die unzureichenden mechanischen Eigenschaften, wie Festigkeit und Duktilität Reines Magnesium und dessen Legierungen weisen bekanntermaßen einen niedrigen Elastizitätsmodul, sowie eine geringe Dichte und mechanische Eigenschaften auf, die denen des menschlichen Knochens sehr ähneln und damit vorteilhaft für die Knochenheilung eingesetzt werden können. Nachteilig ist allerdings, dass die Abbaurate in biologischen Systemen zu hoch ist und z.T. unter starker Wasserstoffentwicklung erfolgt, sowie die geringe Festigkeit und Verformbarkeit [2], [3], [4].

Reines Eisen bzw. eisenbasierte Legierungen besitzen gegenüber Magnesium und Zink und deren Legierungen in der Regel deutlich vorteilhaftere mechanische Eigenschaften, sowie eine deutlich bessere Verarbeitbarkeit, wobei die Abbaurate von reinem Eisen in biologischen Systemen allerdings zu niedrig ist [2], [3].

Bekannt ist, dass Eisenbasislegierungen aufgrund ihrer hohen Festigkeit, Steifigkeit und der guten Verformbarkeit, gekoppelt mit einer guten mechanischen Integrität bei Degradation, der exzellenten Verarbeitbarkeit und der geringen Kosten eine aussichtsreiche Alternative zu Magnesiumlegierungen darstellen [3], [5], [6]. Derzeit werden Forschung und Entwicklung von eisenbasierten Legierungen für den Einsatz als poröse Knochenersatzmaterialien, sowie als Gefäßstützen vorangetrieben, da für diese Anwendungen das große Eigenschaftspotential zum Tragen kommt, wodurch dünnere Streben und damit ein geringerer Materialeinsatz realisiert werden können [2], [7], [8].

Bekannt ist auch, dass eine Verbesserung der mechanischen Eigenschaften, der Degradationsrate sowie der biologischen Kompatibilität beispielsweise durch Legieren, gezielte Variation des Herstellungsprozesses, Bildung von Verbundwerkstoffen oder Oberflächenmodifikation realisierbar ist [3].

Es ist ebenfalls bekannt, dass aufgrund der Kombination von hoher Festigkeit und guter Verformbarkeit, und aufgrund der für Eisenlegierungen verhältnismäßig hohen Degradationsrate sowie der guten biologischen Kompatibilität, Legierungssysteme auf Fe-Mn-Basis aussichtsreich für den Einsatz als Stentwerkstoff sind [2], [3], [9], [10].

Aus DE 10 2008 005 806 A1 sind Legierungen auf Fe-Mn-Basis bekannt, die als weitere Legierungselemente immer Al, N und C gemeinsam enthalten.

US 2013 / 0103161 A1 offenbart Stents aus eisenbasierten Legierungen der Formel Fe-X-Y, mit mindestens einem Element der Gruppe X = Co, Ni, Mn, Cu, Re, Rh, Ru, Ir, Pt, Pd, C und N sowie mindestens einem Element der Gruppe Y = Au, Pd.

Aus DE 10 2015 204 112 A1 sind biologisch abbaubare Eisenbasislegierungen mit erhöhter Abbaurate der Formel Fe-Mn-C-X mit X = ein oder mehrerer Elemente der Gruppe Bor (B), Schwefel (S), Kobalt (Co), Wolfram (W) bekannt. Eine Fe68,99Mn30C1B0,01-Legierung weist bspw. eine Korrosionsrate von 2 mg / cm² bei 37°C in NaCI-Lösung auf.

Aus CN 102605390 A1 ist ein Verfahren zur Herstellung eines biologisch abbaubaren Fe-Zn Legierungsrohres für Stents durch Galvanoformen bekannt. Ein derartiges Fe-Zn Legierungsmaterial mit einem Zn-Gehalt von 1 bis 40 wt.-% weist eine Degradationsrate von 0,58 bis 0,7 mm / a in simulierter Körperflüssigkeit auf.

CN 110477982 A offenbart eine biologisch abbaubare, antibakterielle, anti-Harnstein Klammer für das Harnsystem und ein Verfahren zu deren Herstellung aus einem Fe-Mn-C-Cu Matrixmaterial mit 15 - 30 wt.-% Mn, 0,8 - 3,0 wt.-% Cu und 0,3 - 1,0 wt.-% C. Derartige Klammern weisen eine Degradationsrate von 0,24 bis 0,41 mm/a in simulierter Harnlösung auf. Aus CN 109811265 A ist eine Fe-Mn-Cu-C-Legierung und dessen Verwendung als Implantat mit 10 - 35 wt.-% Mn, 0,2 - 3,0 wt.-% Cu und 0,2 - 1,5 wt.-% C bekannt. Eine derartige Legierung weist Degradationsraten von 0,1 bis 0,3 mm / a in physiologischer Kochsalzlösung auf.

In EP 2 399 620 B1 ist ein Implantat, dessen Körper überwiegend einen Werkstoff mit dem Hauptbestandteil Eisen aufweist, offenbart. Der Werkstoff enthält Schwefel mit einer Konzentration von mehr als 0,2 Gew.-% bis 1 Gew.-%, und mindestens ein Element der Gruppe Calcium, Mangan und Magnesium, wobei die Konzentration von Calcium mindestens 0,1 Gew.-% bis 1 Gew.-%, von Mangan mindestens 0,5 Gew.-% bis 3 Gew.-% und/oder die Konzentration von Magnesium mindestens 0,3 Gew.-% bis 1 Gew.-% beträgt.

Aufgabe der Erfindung ist es, einen Implantatwerkstoff, umfassend eine biologisch abbaubare Eisenbasislegierung, bereitzustellen, der antibakteriell wirksam ist und eine erhöhte Abbaurate in biologischen Systemen aufweist. Weiterhin ist es Aufgabe der Erfindung, die Verwendung eines derartigen Implantatwerkstoffs anzugeben.

Die Aufgabe wird gelöst durch einen Implantatwerkstoff mit den Merkmalen gemäß Anspruch 1, vorteilhafte Ausgestaltungen geben die Unteransprüche wieder. Weiterhin wird die Aufgabe gelöst durch die Verwendung des Implantatwerkstoffs gemäß den Ansprüchen 7 und 8.

Erfindungsgemäß umfasst ein Implantatwerkstoff eine biologisch abbaubare Eisenbasislegierung mit einer Zusammensetzung gemäß Formel (I) Fe_{100-b-c-d-e-f}Mn_{b}C_{c}X_{d}YₑZ_{f}. Dabei ist X = Cu, und Y = Zn und Z = mindestens eines der Elemente Mg, Ca, S. Weiterhin erfindungsgemäß enthält die Eisenbasislegierung mindestens eines der Elemente X, Y und Z und 10 ≤ b ≤ 40 und 0,1 ≤ c ≤ 1,5 und 0 ≤ d ≤ 20 und 0 ≤ e ≤ 30 und 0 ≤ f ≤ 20 (b, c, d, e, f in Massenanteilen).

Vorteilhaft ist ein derartiger Implantatwerkstoff antibakteriell wirksam, biokompatibel und weist eine erhöhte Abbaurate im Vergleich zu bekannten FeMn-Legierungen in biologischen Systemen auf. Biokompatibel im Sinne der Erfindung meint dabei, dass der Implantatwerkstoff nicht krebserregend ist, keine toxischen, allergischen oder zellverändernden Wirkungen im menschlichen Organismus und nur geringe Reaktionen des Immunsystems hervorruft. Antibakteriell wirksam im Sinne der Erfindung meint, dass der Implantatwerkstoff die Vermehrungsfähigkeit und/oder Infektiosität von Bakterien reduziert oder inaktiviert.

In Ausführungsformen umfasst der Implantatwerkstoff Beschichtungen, oberflächennahe Grenzschichten und/oder ist mit mindestens einem weiteren Werkstoff im Sinne eines Verbundwerkstoffs verbunden. Vorteilhaft können dadurch die Eigenschaften des Implantatwerkstoffs in weiten Bereichen variiert werden.

In Ausführungsformen weist der Implantatwerkstoff eine erhöhte Abbaurate gegenüber Fe-Mn-C-Legierungen in biologischen Systemen auf. Die Abbaurate, auch bezeichnet als Korrosionsrate *MR* kann dabei aus der gemessenen Korrosionsstromdichte *i_{corr}* nach ASTM G 102-89(2015) berechnet werden.

Die Korrosionsstromdichte wird mittels des potentiodynamischen Polarisationstests in 150 mM NaCI-Lösung bei 37°C und einem pH-Wert von 7,4 ermittelt.

In Ausführungsformen weist die Eisenbasislegierung eine Korrosionsstromdichte von mindestens 60 µA·cm⁻² auf.

In Ausführungsformen weist die Eisenbasislegierung eine Streckgrenze von mindestens 340 MPa, bevorzugt von mindestens 385 MPa, auf.

In weiteren Ausführungsformen weist die Eisenbasislegierung eine Zugfestigkeit von mindestens 700 MPa, bevorzugt von mindestens 750 MPa, besonders bevorzugt von mindestens 800 MPa, auf.

In weiteren Ausführungsformen weist eine erfindungsgemäße Eisenbasislegierung eine Bruchdehnung von mindestens 25 %, bevorzugt von mindestens 40 %, auf.

In weiteren Ausführungsformen wird die Eisenbasislegierung wärmebehandelt. Dadurch werden vorteilhaft die mechanischen Eigenschaften der Eisenbasislegierung an spezielle Anforderungen des Implantatwerkstoffs im Einsatzfall angepasst.

In Ausführungsformen enthält die Eisenbasislegierung das Element X = Cu oder das Element Y = Zn oder das Element Z = mindestens eines der Elemente Ca, Mg und S. Dabei kann Ca und/oder Mg und/oder S in der Eisenbasislegierung enthalten sein.

In Ausführungsformen enthält die Eisenbasislegierung mindestens das Element Z mit 0,01 ≤ f ≤ 20. Vorteilhaft weist ein derartiger Implantatwerkstoff eine erhöhte Biokompatibilität auf.

In Ausführungsformen enthält die Eisenbasislegierung die Elemente X = Cu mit 0,1 < d ≤ 3 und weiterhin das Element Y = Zn und/oder Z = mindestens eines der Elemente Ca, Mg und S mit 0 ≤ e ≤ 30 und/oder 0 ≤ f ≤ 20. Vorteilhaft ist ein derartiger Implantatwerkstoff antibakteriell wirksam, weist eine erhöhte Biokompatibilität und eine erhöhte Abbaurate in biologischen Systemen auf.

In Ausführungsformen enthält die Eisenbasislegierung die Elemente X = Cu und Y = Zn. Vorteilhaft wird dadurch eine antibakterielle Wirkung erzielt. Weiterhin vorteilhaft bilden sich Cu- und Zn-reiche Zweitphasen aus, welche die Abbaurate der Eisenbasislegierung weiter erhöhen. In Ausführungsformen enthält die Eisenbasislegierung die Elemente X = Cu und Z = mindestens eines der Elemente Ca, Mg und S. Dabei kann Ca und/oder Mg und/oder S in der Eisenbasislegierung enthalten sein. Vorteilhaft wird dadurch eine antibakterielle Wirkung und die Ausbildung einer edlen Cu-reichen Zweitphase erzielt, welche die Abbaurate der Eisenbasislegierung weiter erhöht. Weiterhin vorteilhaft erhöhen die Elemente Ca, Mg und S die Biokompatibilität. Vorteilhaft tragen die Elemente Ca und Mg zur Ausbildung von Lokalelementen und damit zur weiteren Erhöhung der Abbaurate bei.

In Ausführungsformen enthält die Eisenbasislegierung die Elemente X = Cu, Y = Zn und Z = mindestens eines der Elemente Ca, Mg und S. Dabei kann Ca und/oder Mg und/oder S in der Eisenbasislegierung enthalten sein. Vorteilhaft wird damit eine antibakterielle Wirkung und die Ausbildung von Cu- und Zn-reichen Zweitphasen erzielt, welche die Abbaurate der Eisenbasislegierung weiter erhöhen. Weiterhin vorteilhaft erhöhen die Elemente Ca, Mg und S die Biokompatibilität. Vorteilhaft tragen die Elemente Ca und Mg zur Ausbildung von Lokalelementen und damit zur weiteren Erhöhung der Abbaurate bei.

Erfindungsgemäß enthält die Eisenbasislegierung mindestens das Element X, wobei 3 < d ≤ 20 ist, oder die Eisenbasislegierung enthält mindestens das Element Y und/oder Z, wobei 0 ≤ e ≤ 30 und/oder 0 ≤ f ≤ 20 sind.

Vorteilhaft ist eine Eisenbasislegierung, die mindestens das Element X mit 3 < d ≤ 20 enthält, antibakteriell wirksam. Weiterhin vorteilhaft bildet das Element Cu eine edlere, feine Cu-reiche Zweitphase aus, die die Abbaurate der erfindungsgemäßen Eisenbasislegierung erhöht. Weiterhin vorteilhaft weist eine Eisenbasislegierung, die mindestens das Element X mit 3 < d ≤ 20 enthält, eine höhere Korrosionsrate und eine höhere Streckgrenze auf.

In Ausführungsformen weist bspw. eine Eisenbasislegierung enthaltend mindestens das Element X = Cu, mit d = 5, eine Korrosionsstromdichte von mindestens 100 µA·cm⁻² auf.

In Ausführungsformen weist die Eisenbasislegierung enthaltend mindestens das ElementX = Cu mit d = 10 eine Korrosionsstromdichte von mindestens 115 µA·cm⁻² auf.

In Ausführungsformen enthält die Eisenbasislegierung mindestens das Element X = Cu mit3 < d ≤ 5. Vorteilhaft weist eine derartige Eisenbasislegierung ein möglichst homogenes Gefüge auf. Dadurch wird vorteilhaft ein gleichmäßiges Korrosionsverhalten erzielt. Ein möglichst homogenes Gefüge im Sinne der Erfindung umfasst ein austenitisches Gefüge mit homogen verteilten Ausscheidungen und/oder ε-martensitischer Phase.

In weiteren Ausführungsformen enthält die Eisenbasislegierung mindestens das Element X = Cu mit 5 < d ≤ 8. Vorteilhaft weist eine derartige Eisenbasislegierung ein austenitisches Gefüge mit Cu-reichen Ausscheidungen auf.

Weiterhin vorteilhaft weist eine Eisenbasislegierung, die mindestens das Element Y und/oder Z mit 0 ≤ e ≤ 30 und/oder 0 ≤ f ≤ 20 enthält, eine verbesserte Biokompatibilität und/oder eine verbesserte Abbaurate auf.

In bevorzugten Ausführungsformen enthält die Eisenbasislegierung mindestens das Element Y und/oder Z, wobei 0 ≤ e ≤ 30 und/oder 0 ≤ f ≤ 20 sind und weiterhin das Element X, wobei 0,1 < d ≤ 20 ist.

Vorteilhaft ist eine derartige Eisenlegierung antibakteriell wirksam, weist eine erhöhte Abbaurate in biologischen Systemen und eine erhöhte Biokompatibilität auf.

In Ausführungsformen enthält die Eisenbasislegierung mindestens das Element Z = mindestens eines der Elemente Ca, Mg, S mit 0,01 ≤ f ≤ 3, bevorzugt 0,01 ≤ f ≤ 1.

In Ausführungsformen enthält die Eisenbasislegierung mindestens das Element Z, wobei Z = S mit 0,01 ≤ f ≤ 0,5.

In Ausführungsformen enthält die Eisenbasislegierung mindestens das Element Z, wobei Z = Ca oder Mg ist mit 0,1 ≤ f ≤ 1, bevorzugt 0,1 ≤ f ≤ 0,5.

In bevorzugten Ausführungsformen enthält die Eisenbasislegierung herstellungsbedingte Verunreinigungen.

Derartige Verunreinigungen sind unvermeidbar in der Eisenbasislegierung enthalten.

In bevorzugten Ausführungsformen enthält die Eisenbasislegierung herstellungsbedingte Verunreinigungen bis maximal 0,005 Massenanteilen.

In bevorzugten Ausführungsformen sind als herstellungsbedingte Verunreinigungen O, N, und/oder P enthalten.

In bevorzugten Ausführungsformen weist die Eisenbasislegierung ein überwiegend austenitisches Gefüge auf.

Überwiegend austenititisches Gefüge meint, dass die Eisenbasislegierung ein austenitisches Gefüge aufweist, wobei Zweitphasen, bspw. Cu- und/oder Zn-reiche Zweitphasen, sulfidische Ausscheidungen, ferritische und ε-martensitische Phasen, enthalten sein können.

In Ausführungsformen weist ein überwiegend austenitisches Gefüge einen Anteil von Austenit von mindestens 60 Vol.-%, bevorzugt mindestens 80 Vol.-%, besonders bevorzugt von mindestens 90 Vol.-% auf.

In bevorzugten Ausführungsformen wird der erfindungsgemäße Implantatwerkstoff in kardiovaskulären, ösophagealen, urologischen und/oder orthopädischen Implantaten verwendet.

Kardiovaskuläre Implantate meint Implantate zur Anwendung in Herz und Gefäßen, wie bspw. Stents.

Ösophageale Implantate meint Implantate zur Anwendung in der Speiseröhre bspw. Stents.

Urologische Implantate meint Implantate das Harnsystem betreffend bspw. Klammern, Stents.

Orthopädische Implantate meint Implantate zur Anwendung im Stütz- und Bewegungsapparat, wie bspw. dünne Drähte zur Fixierung von Knochen, poröse Implantate als Knochenersatz.

In bevorzugten Ausführungsformen wird ein erfindungsgemäßer Implantatwerkstoff in gießtechnischen, umformenden, pulvermetallurgischen und/oder additiven Fertigungsverfahren zur Herstellung eines Implantats verwendet.

Für die Realisierung der Erfindung ist es auch zweckmäßig, die vorbeschriebenen Ausführungsformen und Merkmale der Ansprüche zu kombinieren.

Nachfolgend soll die Erfindung anhand einiger Ausführungsbeispiele und zugehöriger Figuren eingehender erläutert werden. Die Ausführungsbeispiele sollen dabei die Erfindung beschreiben ohne diese zu beschränken.

### Ausführungsbeispiel

Ein Implantatwerkstoff umfasst eine Eisenbasislegierung der Zusammensetzung

Fe_{100-b-c-d-e-f}Mn_{b}C_{c}X_{d}YₑZ_{f}, (I)

mit X = Cu, und b = 30, c = 1 und d = 5 Massenanteilen.

Die Eisenbasislegierung FeMn30Cu5C1 wird in einem Induktionsofen erschmolzen und in eine zylinderförmige Kupferkokille abgegossen. Bei mittleren Erstarrungsraten von 100 K/ s bis 200 K / s bildet sich ein austenitisches Gefüge mit einer feinen, edleren, Cu-reichen Zweitphase aus. Die Eisenbasislegierung weist eine Festigkeit von 770 MPa und eine Streckgrenze von mindestens 390 MPa auf. Die Eisenbasislegierung weist eine hohe Korrosionsstromdichte von mindestens 100 µA / cm² in potentiodynamischen Polarisationstests in 150 mM NaCl-Lösung bei pH 7,4 auf. Damit ist die Korrosionsstromdichte der FeMn30Cu5C1-Legierung gegenüber einer FeMn30C1-Legierung ohne Cu mit etwa 40 µA / cm² (gemessen in 150 mM NaCI-Lösung bei pH = 7,4) deutlich erhöht.

### Zitierte Nicht-Patentliteratur

[1] H. Hermawan, Updates on the research and development of absorbable metals for biomedical applications, Prog. Biomater. 7 (2018) 93-110. doi: 10.10071540204-018-0091-4
[2] H Hermawan, Biodegradable Metals. From Concept to Application, Springer Berlin Heidelberg, 2012. doi: 10.1007/978-3-642-31170-3
[3] Y.F. Zheng, X.N. Gu, F. Witte, Biodegradable metals, Mater. Sci. Eng. R Reports. 77 (2014) 1-34. doi: 10.1016/j.mser.2014.01.001
[4] N. Li, Y. Zheng, Novel Magnesium Alloys Developed for Biomedical Application: A Review, J. Mater. Sci. Technol. 29 (2013) 489-502. doi: 10.1016/j.jmst.2013.02.005
[5] M. Schinhammer, A.C. Hänzi, J.F. Löffler, P.J. Uggowitzer, Design strategy for biodegradable Fe based alloys for medical applications, Acta Biomater. 6 (2010) 1705-1713. doi: 10.1016/j.actbio.2009.07.039.
[6] O. Bouaziz, S. Allain, C.P. Scott, P. Cozy, D. Barbier, High manganese austenitlc twinning induced plasticity steels: A review of the microstructure properties relationships, Curr. Opin. Solid State Mater. Sci. 15 (2011) 141-168. doi: 10.3016/j.cossms.2011.04.002.
[7] H. Hermawan, D. Dube, D. Mantovani, Developments in metallic biodegradable stents, Acta Biomater. 6 (2010) 1693-1697. doi: 10.1016/j.actbio.2009.10.006.
[8] S.; Loffredo, C. Paternoster, N. Giguere, M. Vedani, D. Mantovani, Effect of Silver on Corrosion Behavior of Plastically Deformed Twinning-induced Plasticity Steel for Biodegradable Stents, Jom , (2020). doi: 10.1007/511837-020-04111-w.
[9] B. Liu, Y. F. Zheng, L. Ruan, In vitro investigation of Fe30Mn6Si shape memory alloy as potential biodegradable metallic material, Mater. Lett. 65 (2011) 540-543 doi:10.1016/j.rnatlet.2010.10.
[10] Gebert, A. et al.,"Corrosion studies on Fe-30Mn-1C alloy in chloride-containing solutions with view to biomedical application", arXiv.org, e-Print Archive, Physics (2019), S.1-28

## Patentansprüche

1. Implantatwerkstoff, umfassend eine biologisch abbaubare Eisenbasislegierung mit einer Zusammensetzung gemäß Formel (I)
Fe_{100-b-c-d-e-f}Mn_{b}C_{c}X_{d}YₑZ_{f}, (I)
wobei X = Cu, und
wobei Y = Zn, und
wobei Z = mindestens eines der Elemente Mg, Ca, S sind, und
wobei die Eisenbasislegierung mindestens eines der Elemente X, Y und Z enthält, und
wobei 10 ≤ b ≤ 40 und 0,1 ≤ c ≤ 1,5 und 0 ≤ d ≤ 20 und 0 ≤ e ≤ 30 und 0 ≤ f ≤ 20 (b, c, d, e, f in Massenanteilen) und
wobei die Eisenbasislegierung mindestens das Element X enthält und 3 < d ≤ 20 ist, oder dass die Eisenbasislegierung mindestens das Element Y und/oder Z enthält

2. Implantatwerkstoff nach Anspruch 1, **dadurch gekennzeichnet, dass** die Eisenbasislegierung mindestens das Element Y und/oder Z enthält, wobei 0≤ e ≤ 30 und/oder 0 ≤ f ≤ 20 sind und weiterhin das Element X enthält und 0,1 < d ≤ 3 ist.

3. Implantatwerkstoff nach einem der Ansprüche 1 oder 2, **dadurch gekennzeichnet, dass** die Eisenbasislegierung herstellungsbedingte Verunreinigungen enthält.

4. Implantatwerkstoff nach Anspruch 3, **dadurch gekennzeichnet, dass** herstellungsbedingte Verunreinigungen bis maximal 0,005 Massenanteilen enthalten sind.

5. Implantatwerkstoff nach Anspruch 3 oder 4, **dadurch gekennzeichnet, dass** als herstellungsbedingte Verunreinigungen O, N, und/oder P enthalten sind.

6. Implantatwerkstoff nach einem der Ansprüche 1 bis 5, **dadurch gekennzeichnet, dass** die Eisenbasislegierung ein überwiegend austenitisches Gefüge aufweist.

7. Verwendung eines Implantatwerkstoffs nach einem der Ansprüche 1 bis 6 in kardiovaskulären, ösophagealen, urologischen und/oder orthopädischen Implantaten.

8. Verwendung eines Implantatwerkstoffes nach einem der Ansprüche 1 bis 7 in gießtechnischen, umformenden, pulvermetallurgischen und/oder additiven Fertigungsverfahren zur Herstellung eines Implantats.
